# EUROPEAN PATENT SPECIFICATION

(11) **EP 0 456 157 B1**
(45) Date of publication and mention of the grant of the patent: **24.08.1994**
(21) Application number: 91107313.8
(22) Date of filing: 06.05.1991
(51) Int. Cl.: C07C 43/313, C07C 317/16, C07D 319/06, C07C 41/56

(54) **Process of producing chloroacetals**
Verfahren zur Herstellung von Chloracetalen
Procédé de préparation de chloroacétals

(30) Priority: 07.05.1990 JP 117111/90
(43) Date of publication of application: 13.11.1991
(73) Proprietor: KUREHA CHEMICAL INDUSTRY CO., LTD., Chuo-ku Tokyo 103 (JP)
(72) Inventor: Ishizuka, Makoto, Kawaguchi-shi, Saitama-ken (JP); Wakasugi, Takashi, Iwaki-shi, Fukushima-ken (JP)
(74) Representative: Boeters, Hans Dietrich, Dr.

(56) References cited:
- EP-A- 0 081 856
- EP-A- 0 247 233
- EP-A- 0 247 234
- DD-A- 240 371
- DE-B- 1 643 899
- DE-C- 1 235 880
- GB-A- 2 146 016
- US-A- 4 532 338
- PATENT ABSTRACTS OF JAPAN, unexamined applications, C field, vol. 13, no. 115, March 20, 1989; THE PATENT OFFICE JAPANESE GOVERNMENT, page 4 C 578

## Description

### FIELD OF THE INVENTION

This invention relates to a process of producing an acyclic or cyclic chloroacetal from a monochloroacetaldehyde trimer.

### BACKGROUND OF THE INVENTION

Hitherto, as a process of producing chloroacetals, there are known (1) a method of subjecting chloroacetaldehyde and an alcohol to a dehydration reaction with anhydrous calcium chloride as described in West German Patent 1,235,880, (2) a method of reacting α , β-dichloroethyl alkyl ether and an alcohol as described in Japanese Patent Publication (unexamined) No. 63-287739, and (3) a method of obtaining 1,2-dihaloethyl acetate by a halogenation of vinyl acetate and reacting the product with an alcohol as described in British Patent 2,146,016 and U.S. Patent 4,532,338. Also, (4) a method of reacting an acyclic chloroacetal and a dihydric alcohol as a process of producing a cyclic chloroacetal is known as described in Japanese Patent Publication (unexamined) No. 61-166829.

However, in the aforesaid production method (1) using chloroacetaldehyde as the raw material, chloroacetaldehyde having a concentration of at least 80% is required but chloroacetaldehyde is a very unstable compound and can not store for a long period of time. Accordingly, when chloroacetaldehyde is used as the raw material for producing chloroacetal, the production of chloroacetalaldehyde and the production of chloroacetal must be carried out continuously, whereby the production steps become complicated and are industrially disadvantageous.

Also, in the production method (2) of using α , β-dichloroethyl alkyl ether as a raw material, the production of the raw material for the formation of acetal and the production of chloroacetal can be separately carried out but since the reaction is a dehydrochloric acid reaction of the raw material and an alcohol, a dehydrochloric acid agent such as triethylamine, tributylamine, etc., is required. Accordingly, after the reaction, the recovery step of the dehydrochloric acid agent is necessary, which make the prodution step complicated. Also, the method is unsuitable for the production of cyclic acetals.

In the production method (3) of starting the chlorination of vinyl acetate, after chlorinating vinyl acetate in benzene, an alcohol is added thereto to aging overnight, and after separating a benzene layer, the reaction is completed by an azeotropic dehydration. Accordingly, the production faculty is low and a large amount of acetic acid is by-produced, which requires an equipment for recovering it. Thus, the method is disadvantageous as an industrial production method.

Furthermore, in the production method (4) of cyclic acetals using chloroacetals as raw materials, chloroacetal which is the raw material for the method is expensive and further in the case of synthesizing, for example, 2-chloromethyl-1,3-dioxolane which is a 5-membered acetal form chloroacetaldehyde diethyl acetal, there is scarecely difference in boiling point between the raw material and the desired product, therefore the purification of the product is difficult.

### SUMMARY OF THE INVENTION

The invention relates to a novel production process of an acyclic or cyclic chloroacetal made for solving the aforesaid problems in the conventional production processes of chloroacetals.

An object of this invention is to provide a process of producing chloroacetals capable of producing high-pure acyclic or cyclic chloroacetals with a good yield.

Other object of this invention is to provide a process of producing chloroacetals capable of independently carrying out the production step of the raw materials for chloroacetals and the production step of the chloroacetals.

A further object of this invention is to provide a process of producing chloroacetals by a simple production step without need of using a large amount of dehydrochloric acid agent and without need of recovering a large amount of acetic acid by-produced.

As the result of the investigations for attaining the aforesaid objects, the inventors have discovered that the aforesaid objects can be attained by using a chloroacetaldehyde trimer which can stably exist as the raw material for produce the acetal and have succeeded in accomplishing the present invention based on the discovery.

That is, according to an embodiment of this invention, there is provided a process of producing acyclic or cyclic chloroacetals, which comprises reacting a monochloroacetaldehyde trimer and a monohydric or dihydric alcohol having from 1 to 5 carbon atoms in the presence of an acid catalyst or a dehydrating agent.

Furthermore, according to another embodiment of this invention, there is provided a process of producing acyclic or cyclic chloroacetals, which comprises heating a monochloroacetaldehyde trimer in the existence of an acid catalyst to form a monomer thereof and reacting the monomer and a monohydric or dihydric alcohol having from 1 to 5 carbon atoms in the presence of an acid catalyst or a dehydrating agent.

### DETAILED DESCRIPTION OF THE INVENTION

Then, the invention is described in detail.

A monochloroacetaldehyde trimer (hereinafter, "monochloroacetaldehyde" is referred to as "MCA") is a white crystal having a melting point of from 87 °C to 88°C and can be easily obtained by dissolving a liquid containing MCA as the main component in an organic solvent such as hexane, etc., and cyclizing MCA in the presence of sulfuric acid as described in Japanese Patent Publication (unexamined) No. 2-223575.

Since the MCA trimer is a stable compound and can be stored for a long period of time, the MCA trimer may be previously prepared and thus the production of chloroacetals of this invention can be carried out independently from the production of the MCA trimer.

As an aliphatic alcohol for producing an acetal by reacting with the MCA trimer, a monohydric or dihydric alcohol having from 1 to 5 carbon atoms is used and examples thereof are monohydric alcohols such as methanol, ethanol, propanol, n-butanol, sec-butanol, ter-butanol, etc., and glycols such as ethylene glycol, propylene glycol, butylene glycol, etc.

Since the acetal forming reaction is almost quantitatively carried out, it is better to use a stoichiometeric amount of the alcohol.

As the catalyst for reacting the MCA trimer and an alcohol, an organic acid or an inorganic acid such as para-toluenesulfonic acid, an acid ion-exchange resin (e.g. , a perfluorosulfonic acid resin), zeolite, etc., is used. The amount of the acid catalyst being used is from 0.1 to 8% by weight, and preferably from 0.2 to 6% by weight to the amount of the reaction charging liquid (the sum of the MCA trimer and an alcohol).

Also, in place of the acid catalyst, a dehydrating agent can be used. As the dehydrating agent, an anhydride of calcium chloride, magnesium sulfate, sodium sulfate, etc., can be used. The amount of the dehydrating agent is from 10 to 60% by weight, and preferably from 15 to 50% by weight to the reaction charging liquid. If the amount of the dehydrating agent is over 60% by weight, the adsorption of the reactants to the dehydrating agent is increased to reduce the yield. On the other hand, if the amount thereof is less than 10% by weight, the reaction does not sufficiently proceed.

In this invention, the reaction proceeds using each one of the acid catalyst and the dehydrating agent but the use of the acid catalyst is preferred. In particular, in the case of synthetizing 7-membered or 8-membered acetals, since the corresponding glycols are viscous, the use of the acid catalyst is particularly preferred.

For the reaction of this invention, a solvent is usually unnecessary but in the reaction with a viscous glycol, a solvent may be used for improving the stirring effect and carrying out the reaction quickly. In such a case, as solvents, organic solvents, e.g., high-boiling organic solvents such as 1,2-dichlorobenzene, etc., are preferably used.

The reaction temperature is at least 120°C, and preferably from 120°C to 160°C under atmospheric pressure or pressure. The reaction is carried out at such a temperature for 1 to 50 hours, and preferably 2 to 30 hours. If the reaction temperature is lower than 120 °C, the MCA trimer is not sufficiently depolymerized, which is undesirable.

After the reaction is over, by distilling the reaction product, a high-pure desired chloroacetal can be obtained with a high yield.

As described above, an MCA trimer may be used for the reaction with an alcohol as it is but after forming a MCA monomer by heating the MCA trimer in the existence of an acid catalyst to perform the depolymerization thereof, the MCA monomer may be used for the reaction with an alcohol.

An MCA trimer reproduces high-pure MCA by heating the trimer to a temperature of from 120°C to 130°C in the existence of an acid catalyst such as diluted sulfuric acid, para-toluenesulfonic acid, etc. After forming MCA, by reacting MCA with an alcohol in the existence of an acid catalyst or a dehydrating agent as in the case of the MCA trimer, a chloroacetal is obtained but in this case, the reaction temperature is at least 30°C, and preferably from 40°C to the refluxing temperature. The process of this invention includes the process of reproducing MCA from an MCA trimer and reacting the MCA with an alcohol as described above.

In each case, acyclic chloroacetals are produced from monohydric alcohols and cyclic chloroacetals are produced from dihydric alcohols.

In addition, MCA obtained by the depolymerization of an MCA trimer which is used for the process of this invention can be stably stored for longer than one week. However, MCA obtained by distilling the chlorinated liquid of para-acetaldehyde is liable to cause a polycondensation and after storing the MCA for one day, the amount of the MCA is reduced to a half. Accordingly, in the conventional technique, it is difficult to separate the production of MCA and the reaction of forming an acetal and also by the reaction of forming an acetal, only a product containing a large amount of impurities is obtained.

Then, the invention is explained more practically by the following examples but the invention is not limited to them.

### Example 1

### Synthesis of 2-chloromethyl-1,3-dioxolane:

In a 100 ml 3 neck distillation flask equipped with a thermometer were placed 20.0 g (0.085 mol) of MCA trimer, 15.75 g (0.25 mol) of ethylene glycol, and 1.02 g of para-toluenesulfonic acid and the reaction was carried out at 120°C. After carrying out the reaction for 20 hours, the reaction mixture was distilled under reduced pressure to provide 23.24 g (yield: 75%) of 2-chloromethyl-1,3-dioxolane having a boiling point of from 59.5°C to 61.0°C /29 mmHg, a purity of 99.5%, and showing the following properties.
- IR(neat):: 1150 cm⁻¹, 1050 cm⁻¹ (-O-C-O-), and 760 cm⁻¹ (C-C1)
- ¹H-NMR(60 MHz,CDCl₃):: 3.5 ppm (2H, d, ClCH₂-)
4.0 ppm (4H, m, -O-CH₂)
5.1 ppm (1H, t, O-CH-O)

### Example 2

### Synthesis of 2-chloromethyl-1,3-dioxolane:

In a 100 ml 3 neck distillation flask equipped with a thermometer was placed 27.9 g of an MCA trimer and the trimer was distilled under atmospheric pressure at 120°C in the existence of 0.5 ml of 6 N sulfuric acid. As the result of analyzing the composition of 26.2 g of the distillate thus obtained by gas chromatography, it was confirmed that the product was MCA having a purity of 99.8%.

The synthesis of 2-chloromethyl-1,3-dioxalane was carried out by charging 25.9 g (0.33 mol) of the aforesaid distillate (MCA) in a 100 ml 3 neck distillation flask equipped with a thermometer and a reflux condenser and reacting the distillate with 20.5 g (0.33 mol) of ethylene glycol using 0.23 g of para-toluenesulfonic acid as a catalyst for 7.5 hours at a temperature of from 75 °C to 80°C. The reaction mixture obtained was distilled under reduced pressure to provide 35.2 g (yield: 87%) of 2-chloromethyl-1,3-dioxolan having a purity of 99.5%.

### Example 3

### Synthesis of 2-chloromethyl-1,3-dioxolane:

The same procedure as Example 2 except that anhydrous calcium chloride was used as a dehydrating agent in place of the acid catalyst was followed.

Thus, by reacting 26.9 g (0.34 mol) of MCA obtained from 28.9 g of the MCA trimer with 21.1 g (0.34 mol) of ethylene glycol in the existence of 14.0 g of anhydrous calcium chloride for 5 hours at a temperature of from 70°C to 80°C and after carring out the reaction, the reaction mixture was distilled under reduced pressure to provide 36.9 g (yield: 88%) of 2-chloromethyl-1,3-dioxolan having a purity of 99.1%.

### Example 4

### synthesis of 2-chloromethyl-1,3-dioxane:

In a 100 ml 3 neck distillation flask equipped with a thermometer were placed 15.0 g (0.064 mol) of an MCA trimer and 0.51 g of para-toluenesulfonic acid and the reaction was carried out at 140°C. After initiating the reaction, 14.5 g (0.19 mol) of propylene glycol was added over a period of 30 minutes and after carrying out the reaction for 3 hours, the reaction mixture was distilled under reduced pressure to provide 21.20 g (yield: 81%) of 2-chloromethl-1,3-dioxane having a purity of 99.5%.

### Example 5

### Synthesis of 2-chloromethyl-1,3-dioxane:

As in Example 2, 22.6 g (0.29 mol) of MCA obtained from 24.8 g of an MCA trimer was reacted with 22.1 g (0.29 mol) of propylene glycol using 0.22 g of para-toluenesulfonic acid as a catalyst for 8 hours at 80 °C. After the reaction was over, the reaction mixture was distilled under reduced pressure to provide 36.7 g (yield: 92%) of 2-chlorometyl-1,3-dioxane having a purity of 99.2%.

### Example 6

### Synthesis of chloroacetaldehyde diethylacetal:

As in Example 3, 17.2 g (0.22 mol) of MCA obtained from 18.6 g of an MCA trimer was reacted with 20.3 g (0.44 mol) of ethanol in the existence of 10.0 g of anhydrous calcium chloride for 3 hours at 70 °C . After the reaction was over, the reaction mixture was distilled under reduced pressure to provide 29.5 g (yield: 88%) of chloroacetaldehyde diethylacetal having a boiling point of from 63°C to 65°C/30 mmHg and a purity of 99.4%.

### Example 7

### Synthesis of 2-chloromethyl-1,3-dioxolane:

The same procedure as Example 1 except that a perfluorosulfonic acid resin (Nafion NR-50, trade name, made by Du Pont), which was an acid ion-exchange resin, as a catalyst in place of para-toluenesulfonic acid was followed.

Thus, 20.0 g (0.085 mol) of an MCA trimer, 15.75 g (0. 25 mol) of ethylene glycol, and 0.48 g of Nafion NR-50 were placed in the flask and the reaction was carried out for 25 hours at 130°C. After the reaction was over, the reaction mixture was distilled under reduced pressure to provide 26.63 g (yield: 87%) of 2-chloromethyl-1,3-dioxolan having a purity of 99.8%.

In addition, Nafion NR-50 could be reused after regeneration.

### Example 8

### Synthesis of chloroacetaldehyde diethylacetal:

In an autoclave were charged 15.0 g (0.064 mol) of an MCA trimer, 18.5 g (0.39 mol) of ethanol, and 0.47 g of para-toluenesulfonic acid and the reaction was carried out for 3 hours at 130°C under pressure. After the reaction was over, the reaction mixture was distilled under reduced pressure to provide 24.1 g (yield: 82%) of chloroacetaldehyde diethylacetal having a purity of 99.6%.

As described above, according to the process of this invention, chloroacetals are produced using the MCA trimer which can be stably stored as the raw material for forming the acetals, high-pure chloroacetals can be produced with a good yield independently from the production of MCA.

Also, in the process of this invention, the step of recovering and regenerating a large amount of a dehydrochloric acid agent as in the conventional process of using α , β -dichloroethyl alkyl ether as a raw material becomes unnecessary or the step of recovering a large amount of by-produced acetic acid as in the conventional method of chlorinating vinyl acetate becomes unnecessary, and hence the production step can be simplified.

The chloroacetals obtained are useful as materials for synthesizing medicines or polymers.

## Claims

1. A process of producing an acyclic or cyclic chloroacetal, which comprises reacting a monochloroacetaldehyde trimer and a monohydric or dihydric alcohol having from 1 to 5 carbon atoms in the presence of an acid catalyst or a dehydrating agent.

2. A process of producing an acyclic or cyclic chloroacetal, which comprises heating monochloroacetaldehyde trimer in the presence of an acid catalyst to form the monomer thereof and then reacting the monomer and a monohydric or dihydric alcohol having from 1 to 5 carbon atoms in the presence of an acid catalyst or a dehydrating agent.

3. The process of producing an acyclic or cyclic chloroacetal as in claim 1 or 2, wherein the acid catalyst is at least one kind of a compound selected from para-toluenesulfonic acid, an acid ion-exchange resin, and an acid zeolite.

4. The process of producing an acyclic or cyclic chloroacetal as in claim 1 or 2, wherein the dehydrating agent is at least one kind of an anhydrous inorganic salt selected from anhydrous calcium chloride, anhydrous magnesium sulfate, and anhydrous sodium sulfate.

5. The process of producing an acyclic or cyclic chloroacetal as in claim 1, wherein the reaction of the monochloroacetaldehyde trimer and the monohydric or dihydric alcohol having from 1 to 5 carbon atoms is carried out at a temperature of from 130°C to 160°C.

6. The process of producing an acyclic or cyclic chloroacetal as in claim 2, wherein the reaction of the monochloroacetaldehyde monomer and the monohydric or dihydric alcohol having from 1 to 5 carbon atoms is carried out at a temperature of from 40°C to the refluxing temperature.

## Patentansprüche

1. Verfahren zur Herstellung eines acyclischen oder cyclischen Chloracetals, umfassend
Umsetzung eines Monochloracetaldehydtrimers und eines einwertigen oder zweiwertigen Alkohols mit 1 bis 5 Kohlenstoffatomen in Gegenwart eines Säurekatalysators oder eines Dehydratisierungsmittels.

2. Verfahren zur Herstellung eines acyclischen oder cyclischen Chloracetals, umfassend
Erhitzen eines Monochloracetaldehydtrimers in Gegenwart eines Säurekatalysators, um dessen Monomer zu bilden und anschließend Umsetzung des Monomers und eines einwertigen oder zweiwertigen Alkohols mit 1 bis 5 Kohlenstoffatomen in Gegenwart eines Säurekatalysators oder eines Dehydratisierungsmittels.

3. Verfahren zur Herstellung eines acyclischen oder cyclischen Chloracetals nach Ansprüchen 1 oder 2, wobei der Säurekatalysator wenigstens eine Verbindung ausgewählt unter paraToluolsulfonsäure, saurem Ionenaustauschharz und saurem Zeolith ist.

4. Verfahren zur Herstellung eines acyclischen oder cyclischen Chloracetals nach Ansprüchen 1 oder 2, wobei das Dehydratisierungsmittel wenigstens ein wasserfreies anorganisches Salz ausgewählt unter wasserfreiem Kalziumchlorid, wasserfreiem Magnesiumsulfat und wasserfreiem Natriumsulfat ist.

5. Verfahren zur Herstellung eines acyclischen oder cyclischen Chloracetals nach Anspruch 1, wobei die Umsetzung des Monochloracetaldehydtrimers und des einwertigen oder zweiwertigen Alkohols mit 1 bis 5 Kohlenstoffatomen bei einer Temperatur von 130°C bis 160°C durchgeführt wird.

6. Verfahren zur Herstellung eines acyclischen oder cyclischen Chloracetals nach Anspruch 2, wobei die Umsetzung des Monochloracetaldehydmonomers und des einwertigen oder zweiwertigen Alkohols mit 1 bis 5 Kohlenstoffatomen bei einer Temperatur von 40°C bis zur Rückflußtemperatur durchgeführt wird.

## Revendications

1. Procédé de production d'un chloracétal acyclique ou cyclique, qui comprend faire réagir un monochloracétaldéhyde trimère et un mono- ou di-alcool contenant de 1 à 5 atomes de carbone en présence d'un catalyseur acide ou d'un agent déshydratant.

2. Procédé de production d'un chloracétate acyclique ou cyclique, qui comprend chauffer le monochloroacétaldéhyde trimère en présence d'un catalyseur acide pour former un monomère de celui-ci et, ensuite, à faire réagir le monomère et un mono- ou di-alcool contenant de 1 à 5 atomes de carbone en présence d'un catalyseur acide ou d'un agent déshydratant.

3. Procédé de production d'un chloracétate acyclique ou cyclique selon la revendication 1 ou 2, dans lequel le catalyseur acide est au moins un type de composé choisi parmi l'acide p-toluènesulfonique, une résine acide échangeuse d'ions et une zéolite acide.

4. Procédé de production d'un chloracétal acyclique ou cyclique selon la revendication 1 ou 2, dans lequel l'agent déshydratant est au moins un type d'un sel minéral anhydre choisi parmi le chlorure de calcium anhydre, le sulfate de magnésium anhydre et le sulfate de sodium anhydre.

5. Procédé de production d'un chloracétal acyclique ou cyclique selon la revendication 1, dans lequel on effectue la réaction du monochloracétaldéhyde trimère et du mono- ou di-alcool contenant de 1 à 5 atomes de carbone a une température de 130 à 160 °C.

6. Procédé de production d'un chloracétal acyclique ou cyclique selon la revendication 2, dans lequel on effectue la réaction du monochloracétaldéhyde monomère et du mono- ou di-alcool contenant de 1 à 5 atomes de carbone à une température de 40 °C à la température de reflux.
